# EUROPEAN PATENT SPECIFICATION

(11) **EP 1 768 700 B1**
(45) Date of publication and mention of the grant of the patent: **15.12.2010**
(21) Application number: 05771041.0
(22) Date of filing: 14.07.2005
(51) Int. Cl.: A61K 47/48, A61P 35/00

(54) **CONJUGATES COMPRISING GM-CSF AND A POLYMER**
KONJUGATE ENTHALTEND GM-CSF UND EIN POLYMER
CONJUGUES COMPRENANT GM-CSF ET UN POLYMER

(30) Priority: 16.07.2004 US 588601 P
(43) Date of publication of application: 04.04.2007
(73) Proprietor: Nektar Therapeutics, San Carlos, CA 94070 (US)
(72) Inventor: BOSSARD, Mary, J., Madison, AL 35758 (US); ZHANG, Ping, Madison, AL 35758 (US)
(74) Representative: Baldock, Sharon Claire
(86) International application number: PCT/US2005/025052
(87) International publication number: WO 2006/019950

(56) References cited:
- EP-A- 0 400 472
- EP-A- 0 402 070
- EP-A- 0 727 437
- EP-A- 1 026 171
- EP-A- 1 303 292
- WO-A-01/87925
- WO-A-02/02132
- WO-A-98/32466
- WO-A-03/040211
- WO-A-2004/012773
- WO-A-2004/018698
- WO-A-2004/022630
- WO-A-2004/060406
- WO-A-2005/000360
- US-A- 5 629 384
- US-A- 5 880 255
- US-A1- 2003 114 647
- US-B1- 6 689 351
- SAIFER M G P ET AL: "IMPROVED CONJUGATION OF CYTOKINES USING HIGH MOLECULAR WEIGHT POLY(ETHYLENE GLYCOL): PEG-GM-CSF AS A PROTOTYPE" POLYMER PREPRINTS, AMERICAN CHEMICAL SOCIETY, US, vol. 38, no. 1, 1997, pages 576-577, XP000884661 ISSN: 0032-3934
- MALIK F ET AL: "Polyethylene Glycol (PEG)- modified Granulocyte-Macrophage Colony- stimulating Factor (GM-CSF) with conserved Biological Activity" EXPERIMENTAL HEMATOLOGY, NEW YORK, NY, US, vol. 20, 1992, pages 1028-1035, XP002138685 ISSN: 0301-472X
- DELGADO C ET AL: "COUPLING OF POLY(ETHYLENE GLYCOL) TO ALBUMIN UNDER VERY MILD CONDITIONS BY ACTIVATION WITH TRESYL CHLORIDE: CHARACTERIZATION OF THE CONJUGATE BY PARTITIONING IN AQUEOUS TWO-PHASE SYSTEMS" BIOTECHNOLOGY AND APPLIED BIOCHEMISTRY, ACADEMIC PRESS, US, vol. 12, no. 2, 1990, pages 119-128, XP009010066 ISSN: 0885-4513
- DEMIROGLOU A ET AL: "A NOVEL REACTION SEQUENCE FOR THE COUPLING OF NUCLEOPHILES TO AGAROSE WITH 2,2,2-TRIFLUOROETHANESULFONYL CHLORIDE" ANGEWANDTE CHEMIE. INTERNATIONAL EDITION, WILEY VCH VERLAG, WEINHEIM, DE, vol. 33, no. 1, 1994, pages 120-123, XP000606513 ISSN: 1433-7851

## Description

### FIELD OF THE INVENTION

The present invention relates generally to conjugates comprising a GM-CSF moiety (i.e., a moiety having GM-CSF activity) and a polymer. In addition, the invention relates to compositions comprising the conjugates.

### BACKGROUND OF THE INVENTION

One important function of the human hematopoietic system is the replacement of a variety of white blood cells (including macrophages, neutrophils, and basophils/mast cells), red blood cells (i.e., erythrocytes) and clot-forming cells (e.g., megakaryocytes/platelets). Each of these specialized cells is formed from hematopoietic precursor cells located in the bone marrow. Specific hormone-like glycoproteins called "colony stimulating factors" control the differentiation and maturation of the hematopoietic precursor cells into any one of the number of specialized blood cells.

One such colony stimulating factor is granulocyte macrophage-colony stimulating factor or "GM-CSF." As its name implies, this colony stimulating factor promotes the proliferation and differentiation of white blood cells such as granulocytes and macrophages, although GM-CSF can promote the formation of other cell types as well. GM-CSF is produced by a number of different cell types (including activated T cells, B cells, macrophages, mast cells, endothelial cells and fibroblasts) in response to cytokine, immune and inflammatory stimuli. Native GM-CSF is a glycoprotein of 127 amino acids and can have a variety of molecular weights depending on the extent of glycosylation.

Pharmacologically, GM-CSF has been administered to cancer patients in order to accelerate the replacement of white blood cells that are killed during chemotherapy treatments. With a similar aim to accelerate white blood cell replacement, this colony stimulating factor has been administered to leukemia patients undergoing bone marrow replacement therapy. Additional applications, such as accelerated wound healing, have been proposed. See, for example, U.S. Patent No. 6,689,351.

One drawback associated with current forms of GM-CSF therapy is the frequency of dosing. Because GM-CSF therapy typically requires daily injections, patients dislike the inconvenience and discomfort associated with this regimen. Coupled with the fact that patients require frequent blood testing to determine white blood cells counts (which require trips to a health care practitioner), many patients would prefer an alternative that is less cumbersome and/or involves a reduction in the number of injections.

One proposed solution to these problems has been to provide a prolonged release form of GM-CSF. For example, U.S. Patent No. 5,942,253 describes microspheres of poly(lactic acid-co-glycolic acid) or other biodegradable polymers of GM-CSF. The formation of microspheres, however, can be a complex process, requiring several synthetic steps. Thus, this prolonged release approach suffers from complexities that are ideally avoided.

PEGylation, or the attachment of a poly(ethylene glycol) derivative to a protein, has been described as a means to prolong a protein's *in vivo* half-life, thereby resulting in prolonged pharmacologic activity. For example, U.S. Patent No. 5,880,255 describes a conjugate of GM-CSF and poly(ethylene glycol) formed from a reaction with 2,2,2-trifluoroethanesulfonate derivatized linear monomethoxy poly(ethylene glycol) having a molecular weight of 5,000 Daltons.

Saifer e Al. "Polymer Preprints", vol 38, 1997 pag. 576-577, discloses PEG-GH-CSF conjugates wherein the PEG claims are linear.

Notwithstanding this described conjugate however, there remains a need for other conjugates of GM-CSF possessing, for example, a polymer having a molecular weight greater than 5,000 Daltons, a polymer having a different structure (e.g., a branched and/or forked structure), different attachment sites, site-specific or site-selective attachment sites, and so forth.

Thus, there remains a need in the art to provide additional GM-CSF moiety-polymer conjugates. Among other things, one or more embodiments of the present invention is therefore directed to such conjugates as well as compositions comprising the conjugates and related methods as described herein, which are believed to be new and completely unsuggested by the art.

### SUMMARY OF THE INVENTION

A pharmaceutical composition comprising:
(i) a conjugate comprising a human GM-CSF covalently attached, either directly or through a spacer moiety comprised of one or more atoms, to a water-soluble polymer, having the following structure: 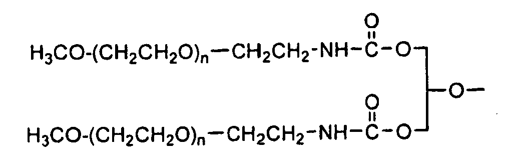 wherein each (n) is independently an integer having a value of from 3 to 4000, and having a weight-average molecular weight of greater than 5,000 Daltons; and
(ii) a pharmaceutically acceptable excipient,
wherein at least about 85% of the conjugates in the composition have from one to two polymers attached to the human GM-CSF.

### BRIEF DESCRIPTION OF THE FIGURES

FIG. 1 is the chromatogram following SEC-HPLC analysis of a conjugate solution as described in Example 1

FIG. 2 is the chromatogram following anion-exchange purification of a composition described in Example 1.

FIG. 3 shows the SDS-PAGE results of conjugate fractions as described in Example 1.

FIG. 4 is the chromatogram following SEC-HPLC analysis of a conjugate solution as described in Example 2.

FIG. 5 is the chromatogram following anion-exchange purification of a composition as described in Example 3.

FIG. 6 is the chromatogram following SEC-HPLC analysis of a conjugate solution as described in Example 4.

FIG. 7 is the chromatogram following SEC-HPLC analysis of a conjugate solution as described in Example 5.

FIG. 8 is the chromatogram following SEC-HPLC analysis of a conjugate solution as described in Example 6.

### DETAILED DESCRIPTION OF THE INVENTION

The GM-CSF Moiety

The GM-CSF moiety can be derived from either non-recombinant methods or from recombinant methods.

The GM-CSF moiety can be derived non-recombinantly. For example, the GM-CSF can be obtained from blood-derived sources. In particular, GM-CSF can be isolated from human plasma or tissues using techniques (e.g., precipitation techniques, centrifugation techniques, chromatographic techniques) known to those of ordinary skill in the art.

The GM-CSF moiety can be derived from recombinant methods. For example, the cDNA coding for human GM-CSF has been isolated, characterized, and cloned into expression vectors. See, e.g., U.S. Patent Nos. 5,078,996 and 5,891,429, and Wong et al. (1985) "Human GM-CSF: Molecular Cloning of the Complementary DNA and Purification of the Natural and Recombinant Proteins," Science 218:819, and Cantrell et al. (1985) "Cloning, Sequence, and Expression of a Human Granulocyte/Macrophage Colony-Stimulating Factor," Proc. Natl. Acad. Sci. U.S.A., Vol. 82: 6250. GM-CSF moieties expressed in bacterial (*Escherichia coli*), mammalian (e.g., Chinese hamster ovary cells), and yeast (e.g., *Saccharomyces cerevisiae*) expression systems can be used.

Once expressed, endogenous human GM-CSF is a monomeric glycoprotein with a molecular weight of about 22,000 Daltons. The expressed amino acid sequence is provided as SEQ ID NO: 1. Preferred for use as a GM-CSF moiety herein are any of a number amino acid sequences of human GM-CSF. At least three different human GM-CSF proteins have been produced in various expression systems: sargramostim; molgramostim; regramostim; and ecogramostim. Sargramostim is expressed in *Saccharomyces cerevisiae,* has an amino acid substitution of leucine at position 23 (SEQ ID NO: 2) as compared to endogenous human GM-CSF and is O-glycosylated. Molgramostim is expressed in *Escherichia coli* and is nonglycosylated. Regramostim is produced in hamster ovary cells (CHO) cells and is fully glycosylated. Methionyl versions of these proteins are also contemplated wherein a methione residue precedes the complete amino acid sequence. Unless specifically noted, all assignments of a numeric location of an amino acid residue as provided herein are based on SEQ ID NO: 1.

Exemplary recombinant methods used to prepare a GM-CSF moiety (whether a human GM-CSF or a different protein having GM-CSF activity) can be briefly described. Such methods involve constructing the nucleic acid encoding the desired polypeptide or fragment, cloning the nucleic acid into an expression vector, transforming a host cell (e.g., plant, bacteria such as *Escherichia coli,* yeast such as *Saccharomyces cerevisiae,* or mammalian cell such as Chinese hamster ovary cell or baby hamster kidney cell), and expressing the nucleic acid to produce the desired polypeptide or fragment. The expression can occur via exogenous expression (when the host cell naturally contains the desired genetic coding) or via endogenous expression. Methods for producing and expressing recombinant polypeptides *in vitro* and in prokaryotic and eukaryotic host cells are known to those of ordinary skill in the art. See, for example, U.S. Patent No. 4,868,122.

To facilitate identification and purification of the recombinant polypeptide, nucleic acid sequences that encode for an epitope tag or other affinity binding sequence can be inserted or added in-frame with the coding sequence, thereby producing a fusion protein comprised of the desired polypeptide and a polypeptide suited for binding. Fusion proteins can be identified and purified by first running a mixture containing the fusion protein through an affinity column bearing binding moieties (e.g., antibodies) directed against the epitope tag or other binding sequence in the fusion proteins, thereby binding the fusion protein within the column. Thereafter, the fusion protein can be recovered by washing the column with the appropriate solution (e.g., acid) to release the bound fusion protein. The recombinant polypeptide can also be identified and purified by lysing the host cells, separating the polypeptide, e.g., by size exclusion chromatography, and collecting the polypeptide. These and other methods for identifying and purifying recombinant polypeptides are known to those of ordinary skill in the art. In one or more embodiments of the present invention, however, it is preferred that the GM-CSF moiety is not in the form of a fusion protein.

Depending on the system used to express proteins having GM-CSF activity, the GM-CSF moiety can be unglycosylated or glycosylated and either may be used. That is, the GM-CSF moiety can be unglycosylated or the GM-CSF moiety can be glycosylated. In one or more embodiments of the invention, it is preferred that the GM-CSF moiety is glycosylated. Examples of glycosylation include O-glycosylation and N-glycosylation. It is believed that the glycosylation sites of endogenous human GM-CSF are serine 9 (O-glycosylation), threonine 10 (O-glycosylation), asparagine 27 (N-glycosylation) and asparagine 37 (N-glycosylation). Preferred glycosylation arrangements of any GM-CSF moiety will occur at these sites (or sites corresponding to these locations on the given GM-CSF moiety). Thus, the GM-CSF moiety can have a degree of glycosylation selected from the group consisting of: no glycosylation, glycosylation at a single site; glycosylation at two sites; glycosylation at three sites; and glycosylation at four sites. A particularly preferred glycosylation arrangement is O-glycosylation only at serine 9 and threonine 10 and without N-glycosylation.

The moiety having GM-CSF activity can advantageously be modified to include one or more amino acid residues such as, for example, lysine, cysteine and/or arginine, in order to provide facile attachment of a polymer to an atom within an amino acid. For example, U.S. Patent No. 6,608,183 describes "cysteine-added" sequences of GM-CSF that can be used as a GM-CSF moiety and methods for preparing such "cysteine-added" sequences. In addition, the GM-CSF moiety can be modified to include a non-naturally occurring amino acid residue. Techniques for adding amino acid residues and non-naturally occurring amino acid residues are well known to those of ordinary skill in the art. See, for example, U.S. Patent No. 5,393,870 and J. March, Advanced Organic Chemistry: Reactions Mechanisms and Structure, 4th Ed. (New York: Wiley-Interscience, 1992).

In addition, the GM-CSF moiety can advantageously be modified to include attachment of a functional group (other than through addition of a functional group-containing amino acid residue). For example, the GM-CSF moiety can be modified to include a thiol group. In addition, the GM-CSF moiety can be modified to include an N-terminal alpha carbon. In addition, the GM-CSF moiety can be modified to include one or more carbohydrate moieties. GM-CSF moieties modified to contain an aminoxy, aldehyde or other functional group can also be used. Furthermore, oxidized variants of GM-CSF can be used as a GM-CSF moiety. See, for example, U.S. Patent No. 5,358,707. Derivatives of GM-CSF are also included as GM-CSF moieties. See U.S. Patent No. 5,298,603.

For any given moiety, it is possible to determine whether that moiety has GM-CSF activity. For example, as described in U.S. Patent No. 5,393,870, human bone marrow from the iliac crest of healthy donors can be collected, placed into solution and centrifuged, with cells being collected and diluted for subsequent culturing. Following culturing, each colony of cells can be identified and the proposed GM-CSF moiety can be added to the appropriate colony and tested for accelerated proliferation relative to a control. Other methods known to those of ordinary skill in the art can also be used to determine whether a given moiety has GM-CSF activity. Such methods are useful for determining the GM-CSF activity of both the moiety itself (and therefore can be used as a "GM-CSF moiety") as well as the corresponding polymer-moiety conjugate.

Examples of GM-CSF moieties include the following: a human GM-CSF as identified in any of SEQ ID NO: 1, SEQ ID NO: 2 and SEQ ID NO: 3; truncated versions thereof; hybrid variants, and peptide mimetics having GM-CSF activity. Biologically active fragments, deletion variants, substitution variants or addition variants of any of the foregoing that maintain at least some degree of GM-CSF activity can also serve as a GM-CSF moiety.

Depending on the system used to express proteins having GM-CSF activity, the GM-CSF moiety can be unglycosylated or glycosylated and either may be used. That is, the GM-CSF moiety can be unglycosylated or the GM-CSF moiety can be glycosylated.

The Water-Soluble Polymer

As previously discussed, each conjugate comprises a GM-CSF attached, either directly or through a spacer moiety comprised of one or more atoms, to a water-soluble polymer according to the claims.

Conjugates

As described above, a conjugate of the invention comprises a water-soluble polymer covalently attached (either directly or through a spacer moiety) to a GM-CSF moiety. Typically, for any given conjugate, there will be one to four water-soluble polymers covalently attached to a GM-CSF moiety (wherein for each water-soluble polymer, the water-soluble polymer can be attached either directly to the GM-CSF moiety or through a spacer moiety). In some instances, however, the conjugate may have 1, 2, 3, 4, 5, 6, 7, 8 or more water-soluble polymers individually attached to a GM-CSF moiety (again, with respect to each water-soluble polymer, attached directly or through a spacer moiety). In addition, the conjugate may include not more than 8 water-soluble polymers individually attached to a GM-CSF moiety, not more than 7 water-soluble polymers individually attached to a GM-CSF moiety, not more than 6 water-soluble polymers individually attached to a GM-CSF moiety, not more than 5 water-soluble polymers individually attached to a GM-CSF moiety, not more than 4 water-soluble polymers individually attached to a GM-CSF moiety, not more than 3 water-soluble polymers individually attached to a GM-CSF moiety, not more than 2 water-soluble polymers individually attached to a GM-CSF moiety, and not more than 1 water-soluble polymer attached to a GM-CSF moiety.

The particular linkage between the GM-CSF moiety and the water-soluble polymer (or the spacer moiety that is attached to the water-soluble polymer) depends on a number of factors. Such factors include, for example, the particular linkage chemistry employed, the particular GM-CSF moiety, the available functional groups within the GM-CSF moiety (either for attachment to a polymer or conversion to a suitable attachment site), the possible presence of additional reactive functional groups within the GM-CSF moiety.

In one or more embodiments of the invention, the linkage between the GM-CSF moiety and the polymer (or the spacer moiety that is attached to the polymer) is a hydrolytically stable linkage, such as an amide, urethane (also known as carbamate), amine, thioether (also known as sulfide), or urea (also known as carbamide). In one or more embodiments, the linkage does not result from reaction of the polymeric reagent bearing a functional group with the GM-CSF moiety, wherein the functional group is selected from the group consisting of triazine, hydrazine, hydrazide, aldehyde, semicarbazide, maleimide, vinylsulfone, phenylglyoxal, isocyanate, isothiocyanate, amine and tresyl functional group with the GM-CSF moiety.

In one or more embodiments of the invention, the linkage between the GM-CSF moiety and the water-soluble polymer (or the spacer moiety that is attached to the water-soluble polymer) is a degradable linkage. In this way, the linkage linking the GM-CSF moiety is "degradable." That is, the water-soluble polymer (and the spacer moiety, when present) cleaves (either through hydrolysis, enzymatic processes, or otherwise), thereby resulting in the native or an unconjugated GM-CSF moiety. Preferably, degradable linkages result in the water-soluble polymer (and any spacer moiety) detaching from the GM-CSF moiety *in vivo* without leaving any fragment of the water-soluble polymer (and any spacer moiety). Exemplary degradable linkages include carbonate, carboxylate ester, phosphate ester, thiolester, anhydrides, acetals, ketals, acyloxyalkyl ether, imines, and orthoesters. Such linkages can be readily prepared by appropriate modification of either the GM-CSF moiety (e.g., the carboxyl group C terminus of the protein or a side chain hydroxyl group of an amino acid such as serine or threonine contained within the protein) and/or the polymeric reagent using coupling methods commonly employed in the art. Most preferred, however, are hydrolyzable linkages that are readily formed by reaction of a suitably activated polymer with a non-modified functional group contained within the GM-CSF moiety.

With regard to linkages, in one more embodiments of the invention, a conjugate is provided, comprising a GM-CSF moiety covalently attached at an amino acid residue, either directly or through a spacer moiety comprised of one or more atoms, to a water-soluble polymer.

The conjugates (as opposed to an unconjugated GM-CSF moiety) may or may not possess a measurable degree of GM-CSF activity. That is to say, a conjugate in accordance with the invention will possesses anywhere from about 0% to about 100% or more of the bioactivity of the unmodified parent GM-CSF moiety. Preferably, compounds possessing little or no GM-CSF activity typically contain a degradable linkage connecting the polymer to the moiety, so that regardless of the lack of activity in the conjugate, the active parent molecule (or a derivative thereof having GM-CSF activity) is released by degradation of the linkage (e.g., hydrolysis upon aqueous-induced cleavage of the linkage). Such activity may be determined using a suitable *in vivo* or *in vitro* model, depending upon the known activity of the particular moiety having GM-CSF activity employed.

Optimally, degradation of a degradable linkage is facilitated through the use of hydrolytically cleavable and/or enzymatically degradable linkages such as urethane, amide, carbonate or ester-containing linkages. In this way, clearance of the conjugate [via cleavage of individual water-soluble polymer(s)] can be modulated by selecting the polymer molecular size and the type of functional group that would provide the desired clearance properties. One of ordinary skill in the art can determine the proper molecular size of the polymer as well as the cleavable functional group. For example, one of ordinary skill in the art, using routine experimentation, can determine a proper molecular size and cleavable functional group by first preparing a variety of polymer-(GM-CSF) conjugates with different weight-average.molecular weights and degradable functional groups, and then obtaining the clearance profile for each conjugate by administering the conjugate to a patient and taking periodic blood and/or urine sampling. Once a series of clearance profiles has been obtained for each tested conjugate, a conjugate having the desired clearance profile can be determined.

For conjugates possessing a hydrolytically stable linkage that couples the GM-CSF moiety to the water-soluble polymer, the conjugate will typically possess a measurable degree of GM-CSF activity. For instance, such conjugates are typically characterized as having a bioactivity satisfying one or more of the following percentages relative to that of the unconjugated GM-CSF moiety: at least about 2%, at least about 5%, at least about 10%, at least about 15%, at least about 25%, at least about 30%, at least about 40%, at least about 50%, at least about 60%, at least about 80%, at least about 85%, at least about 90%, at least about 95%, at least about 97%, at least about 100%, and more than 105% (when measured in a suitable model, such as those presented here and/or well known in the art). Preferably, conjugates having a hydrolytically stable linkage (e.g., an amide linkage) will possess at least some degree of the bioactivity of the unmodified parent GM-CSF moiety.

Exemplary conjugates will now be described. The GM-CSF moiety is expected to share (at least in part) an amino acid sequence similar or related to a human GM-CSF. Thus, as previously indicated, while reference will be made to specific locations or atoms within a human GM-CSF, such a reference is for convenience only and one having ordinary skill in the art will be able to readily determine the corresponding location or atom in other moieties having GM-CSF activity. In particular, the description provided herein for a human GM-CSF is often applicable not only to a human GM-CSF, but to fragments, deletion variants, substation variants and addition variants of any of the foregoing.

Amino groups on GM-CSF moieties provide a point of attachment between the GM-CSF moiety and the water-soluble polymer. Each of the human GM-CSF moieties provided in SEQ ID NO: 1, SEQ ID NO: 2 and SEQ ID NO: 3, comprises 14 lysine residues, each lysine residue containing an ε-amino group that may be available for conjugation, as well as one amino terminus. See SEQ ID NO: 1 and SEQ ID NO: 2 and SEQ ID NO: 3. Thus, exemplary attachment points include attachment at an amino acid (through the amine-containing side chain of a lysine residue) at any one or more of positions 25, 26, 28, 49, 55, 59, 61, 66, 64, 73, 77, 110, 114 and 115. Additionally, another GM-CSF moiety contains 15 amine-containing lysine residues (see SEQ ID NO: 3). Thus, preferred attachment points of this GM-CSF include attachment at the amine residue associated with a lysine at any one of positions 23, 25, 26, 28, 49, 55, 59, 61, 66, 64, 73, 77, 110, 114 and 115.

There are a number of examples of suitable water-soluble polymeric reagents useful for forming covalent linkages with available amines of a GM-CSF moiety. Specific examples, along with the corresponding conjugates, are provided in Table 1, below. In the table, the variable (n) represents the number of repeating monomeric units and "(GM-CSF)" represents the GM-CSF moiety following conjugation to the water-soluble polymer.

**Table 1**

| Amine-Specific Polymeric Reagents and the GM-CSF Moiety Conjugate Formed Therefrom | |
|---|---|
| Polymeric Reagent | Corresponding Conjugate |
| 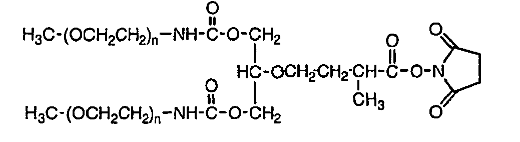 Branched mPEG2-N-Hydroxysuccinimide Reagent | 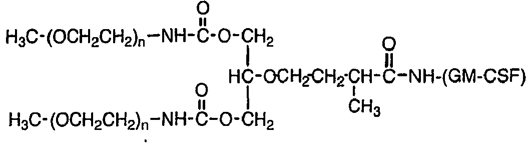 Amide Linkage |
| 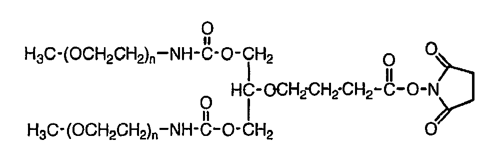 Branched mPEG2-N-Hydroxysuccinimide Reagent | 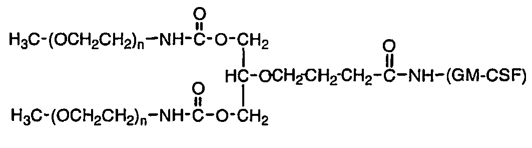 Amide Linkage |
| 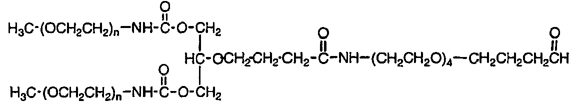 Branched mPEG2 Butyraldehyde Reagent | 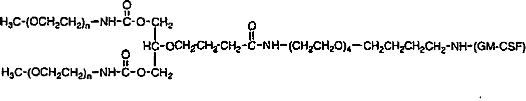 Secondary Amine Linkage |

Conjugation of a polymeric reagent to an amine group of a GM-CSF moiety can be accomplished by a variety of techniques. In one approach, a GM-CSF moiety can be conjugated to a polymeric reagent functionalized with a succinimidyl derivative (or other activated ester group, wherein approaches similar to those described for a succinimidyl derivative can be used for other activated ester group-containing polymeric reagents). In this approach, the polymeric reagent bearing a succinimidyl group can be attached to the GM-CSF moiety in aqueous media at a pH of 7.0 to 9.0, although different reaction conditions (e.g., a lower pH such as 6 to 7, or different temperatures and/or less than 15 °C) can result in the attachment of a polymer to a different location on the GM-CSF moiety.

Typical of another approach useful for conjugating a GM-CSF moiety to a polymeric reagent is the use of a reductive amination reaction to conjugate a primary amine of a GM-CSF moiety with a polymeric reagent functionalized with a ketone, aldehyde or a hydrated form thereof (e.g., ketone hydrate and aldehyde hydrate). In this approach, the primary amine from the GM-CSF moiety reacts with the carbonyl group of the aldehyde or ketone (or the corresponding hydroxy-containing group of a hydrated aldehyde or ketone), thereby forming a Schiff base. The Schiff base, in turn, can then be reductively converted to a stable conjugate through use of a reducing agent such as sodium borohydride. Selective reactions (e.g., at the N-terminus are possible) are possible, particularly with a polymer functionalized with a ketone or an alpha-methyl branched aldehyde and/or under specific reaction conditions (e.g., reduced pH).

Exemplary conjugates that can be prepared using, for example, polymeric reagents containing an aldehyde (or aldehyde hydrate) or ketone or (ketone hydrate) comprise the following structure: 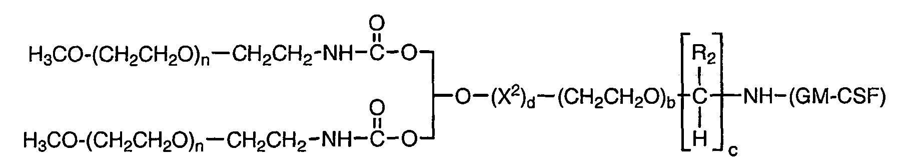 wherein:
each (n) is independently an integer having a value of from 3 to 4000;
X² is a spacer moiety comprised of one or more atoms
(b) is 2 through 6;
(c) is 2 through 6;
R², in each occurrence, is independently H or lower alkyl; and
GM-CSF is a GM-CSF moiety.

Carboxyl groups represent another functional group that can serve as a point of attachment on the GM-CSF moiety.

As discussed above, the specific linkage will depend on the type of functional group utilized. If the polymer is end-functionalized or "activated" with a hydroxyl group, the resulting linkage will be a carboxylic acid ester and X will be O. If the polymer backbone is functionalized with a thiol group, the resulting linkage will be a thioester and X will be S. When certain multi-arm, branched or forked polymers are employed, the C(O)X moiety, and in particular the X moiety, may be relatively more complex and may include a longer linkage structure.

Polymeric reagents containing a hydrazide moiety are also useful for conjugation at a carbonyl. To the extent that the GM-CSF moiety does not contain a carbonyl moiety, a carbonyl moiety can be introduced by reducing any carboxylic acids (e.g., the C-terminal carboxylic acid) and/or by providing a glycosylated version (wherein the added sugar has a carbonyl moiety) of the GM-CSF moiety. In addition, any polymeric reagent comprising an activated ester (e.g., a succinimidyl group) can be converted to contain a hydrazide moiety by reacting the polymeric reagent comprising the activated ester with hydrazine (NH₂-NH₂) or tert-butyl carbazate [NH₂NHCO₂C(CH₃)₃]. In the table, the variable (n) represents the number of repeating monomeric units and "=C-(GM-CSF)" represents a GM-CSF moiety following conjugation to the polymeric reagent. Optionally, the hydrazone linkage can be reduced using a suitable reducing agent.

Thiol groups contained within the GM-CSF moiety can serve as effective sites of attachment for the water-soluble polymer. The thiol groups in cysteine residues of the GM-CSF moiety can be reacted with an activated PEG that is specific for reaction with thiol groups, e.g., an N-maleimidyl polymer or other derivative, as described in, for example, U.S. Patent No. 5,739,208, in International Patent Publication No. WO 01/62827, and in Table 3 below.

With respect to both SEQ ID NO: 1, SEQ ID NO: 2, SEQ ID NO: 3, there are four thiol-containing cysteine residues. It is believed that all cysteine residues in these sequences are involved with disulfide bonding. As a consequence, conjugation to a cysteine residue participating in disulfide bonding may disrupt the tertiary structure of a GM-CSF moiety and potentially significantly decrease its overall activity. Thus, to the extent that any particular GM-CSF moiety lacks a thiol group or disruption of disulfide bonds is to be avoided, it is possible to add a cysteine residue to the GM-CSF moiety using conventional synthetic techniques. See, for example, U.S. Patent No. 6,608,183 and the procedure described in International Patent Publication WO 90/12874, wherein such a procedure can be adapted for a GM-CSF moiety. In addition, conventional genetic engineering processes can also be used to introduce a cysteine residue into the GM-CSF moiety.

With respect to conjugates formed from water-soluble polymers bearing one or more maleimide functional groups (regardless of whether the maleimide reacts with an amine or thiol group on the GM-CSF moiety), the corresponding maleamic acid form(s) of the water-soluble polymer can also react with the GM-CSF moiety. Under certain conditions (e.g., a pH of about 7-9 and in the presence of water), the maleimide ring will "open" to form the corresponding maleamic acid. The maleamic acid, in turn, can react with an amine or thiol group of a GM-CSF moiety. Exemplary maleamic acid-based reactions are schematically shown below. POLY represents the water-soluble polymer, and GM-CSF represents the GM-CSF moiety.

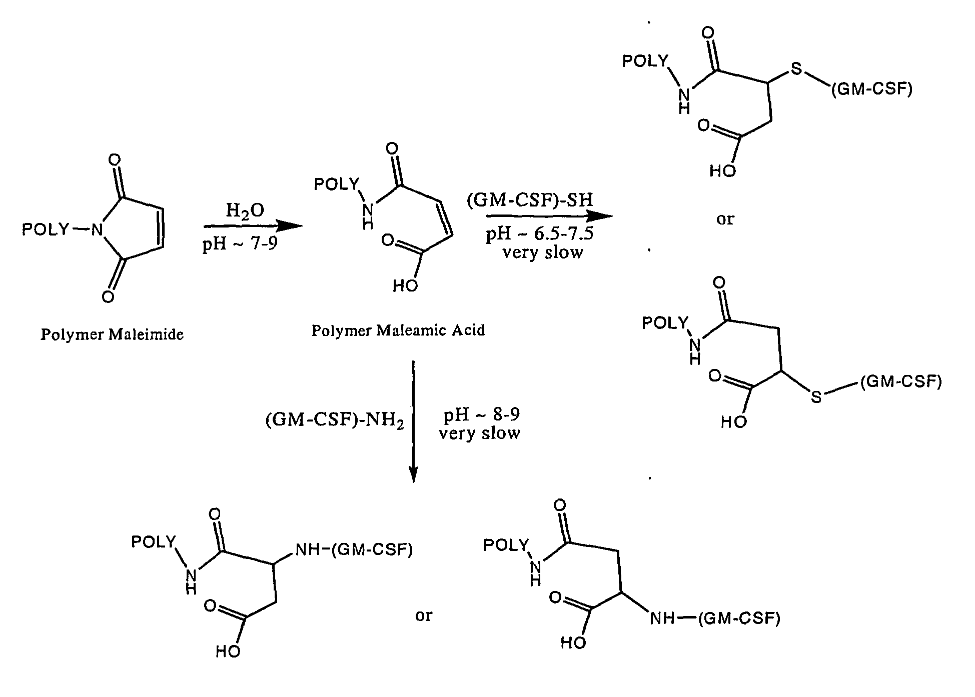

With respect to polymeric reagents, those described here and elsewhere can be purchased from commercial sources (e.g., Nektar Therapeutics, Huntsville AL). In addition, methods for preparing polymeric reagents are described in the literature.

The attachment between the GM-CSF moiety and water-soluble polymer can be direct, wherein no intervening atoms are located between the GM-CSF moiety and the polymer, or indirect, wherein one or more atoms are located between the GM-CSF moiety and polymer. With respect to the indirect attachment, a "spacer moiety" serves as a link between the GM-CSF moiety and the water-soluble polymer. The one or more atoms making up the spacer moiety can include one or more of carbon atoms, nitrogen atoms, sulfur atoms, oxygen atoms, and combinations thereof. The spacer moiety can comprise an amide, secondary amine, carbamate, thioether, and/or disulfide group. Examples of specific spacer moieties (including "X", X¹ and X²) include those selected from the group consisting of -O-, -S-, -S-S-, -C(O)-, -C(O)-NH-, -NH-C(O)-NH-, -O-C(O)-NH-, -C(S)-, -CH₂-, -CH₂-CH₂-, -CH₂-CH₂-CH₂-, -CH₂-CH₂-CH₂-CH₂-, -O-CH₂-, -CH₂-O-, -O-CH₂-CH₂-, -CH₂-O-CH₂-, -CH₂-CH₂-O-, -O-CH₂-CH₂-CH₂-, -CH₂-O-CH₂-CH₂-, -CH₂-CH₂-O-CH₂-, -CH₂-CH₂-CH₂-O-, -O-CH₂-CH₂-CH₂-CH₂-, -CH₂-O-CH₂-CH₂-CH₂-, -CH₂-CH₂-O-CH₂-CH₂-, -CH₂-CH₂-CH₂-O-CH₂-, -CH₂-CH₂-CH₂-CH₂-O-, -C(O)-NH-CH₂-, -C(O)-NH-CH₂-CH₂-, -CH₂-C(O)-NH-CH₂-, -CH₂-CH₂-C(O)-NH-, -C(O)-NH-CH₂-CH₂-CH₂-, -CH₂-C(O)-NH-CH₂-CH₂-, -CH₂-CH₂-C(O)-NH-CH₂-, -CH₂-CH₂-CH₂-C(O)-NH-, -C(O)-NH-CH₂-CH₂-CH₂-CH₂-, -CH₂-C(O)-NH-CH₂-CH₂-CH₂-, -CH₂-CH₂-C(O)-NH-CH₂-CH₂-, -CH₂-CH₂-CH₂-C(O)-NH-CH₂-, -CH₂-CH₂-CH₂-C(O)-NH-CH₂-CH₂-, -CH₂-CH₂-CH₂-CH₂-C(O)-NH-, -C(O)-O-CH₂-, -CH₂-C(O)-O-CH₂-, -CH₂-CH₂-C(O)-O-CH₂-, -C(O)-O-CH₂-CH₂-, -NH-C(O)-CH₂-, -CH₂-NH-C(O)-CH₂-, -CH₂-CH₂-NH-C(O)-CH₂-, -NH-C(O)-CH₂-CH₂-, -CH₂-NH-C(O)-CH₂-CH₂-, -CH₂-CH₂-NH-C(O)-CH₂-CH₂-, -C(O)-NH-CH₂-, -C(O)-NH-CH₂-CH₂-, -O-C(O)-NH-CH₂-, -O-C(O)-NH-CH₂-CH₂-, -NH-CH₂-, -NH-CH₂-CH₂-, -CH₂-NH-CH₂-, -CH₂-CH₂-NH-CH₂-, -C(O)-CH₂-, -C(O)-CH₂-CH₂-, -CH₂-C(O)-CH₂-, -CH₂-CH₂-C(O)-CH₂-, -CH₂-CH₂-C(O)-CH₂-CH₂-, -CH₂-CH₂-C(O)-, -CH₂-CH₂-CH₂-C(O)-NH-CH₂-CH₂-NH-, -CH₂-CH₂-CH₂-C(O)-NH-CH₂-CH₂-NH-C(O)-, -CH₂-CH₂-CH₂-C(O)-NH-CH₂-CH₂-NH-C(O)-CH₂-, -CH₂-CH₂-CH₂-C(O)-NH-CH₂-CH₂-NH-C(O)-CH₂-CH₂-, -O-C(O)-NH-[CH₂]ₕ-(OCH2CH2)ⱼ-, bivalent cycloalkyl group, -O-, -S-, an amino acid, -N(R⁶)-, and combinations of two or more of any of the foregoing, wherein R⁶ is H or an organic radical selected from the group consisting of alkyl, substituted alkyl, alkenyl, substituted alkenyl, alkynyl, substituted alkynyl, aryl and substituted aryl, (h) is zero to six, and (j) is zero to 20. Other specific spacer moieties have the following structures: -C(O)-NH-(CH₂)₁₋₆-NH-C(O)-, -NH-C(O)-NH-(CH₂)₁₋₆-NH-C(O)-, and -O-C(O)-NH-(CH₂)₁₋₆-NH-C(O)-, wherein the subscript values following each methylene indicate the number of methylenes contained in the structure, e.g., (CH₂)₁₋₆ means that the structure can contain 1, 2, 3, 4, 5 or 6 methylenes. Additionally, any of the above spacer moieties may further include an ethylene oxide oligomer chain comprising 1 to 20 ethylene oxide monomer units [i.e., -(CH₂CH₂O)₁₋₂₀]. That is, the ethylene oxide oligomer chain can occur before or after the spacer moiety, and optionally in between any two atoms of a spacer moiety comprised of two or more atoms. Also, the oligomer chain would not be considered part of the spacer moiety if the oligomer is adjacent to a polymer segment and merely represent an extension of the polymer segment. The spacer moiety does not include sugars or carbohydrates (that is, a spacer moiety specifically does not include the sugar of a glycosylate residue). The water-soluble polymer can be attached through a glycosylate residue (e.g., a sugar or carbohydrate). In those instances where such an arrangement is desired, the present application will refer to such an arrangement as "a GM-CSF moiety covalently attached to a water-soluble polymer through a glycosylate residue."

Compositions

The conjugates are typically part of a composition. Generally, the composition comprises a plurality of conjugates, preferably although not necessarily, each having one, two, three or four water-soluble polymers separately covalently attached (either directly or through a spacer moiety) to one GM-CSF moiety. The compositions, however, can also comprise other conjugates having four, five, six, seven, eight or more polymers attached to any given GM-CSF moiety.

In one or more embodiments, it is preferred that the conjugate-containing composition is free or substantially free of albumin. It is also preferred that the composition is free or substantially free of proteins that do not have GM-CSF activity.

If desired, conjugates having different molecular weights can be isolated using gel filtration chromatography and/or ion exchange chromatography. That is to say, gel filtration chromatography is used to fractionate differently numbered polymer-to-(GM-CSF) moiety ratios [e.g., 1-mer, 2-mer, 3-mer, and so forth, wherein "1-mer" indicates 1 polymer attached to a GM-CSF moiety (or monoPEGylated when the polymer is PEG), "2-mer" indicates two polymers attached to GM-CSF moiety (or diPEGylated when the polymer is PEG), and so on] on the basis of their differing molecular weights (where the difference corresponds essentially to the average molecular weight of the water-soluble polymer portion). For example, in an exemplary reaction where a 20,000 Dalton protein is randomly conjugated to a PEG reagent having a molecular weight of about 20,000 Daltons, the resulting reaction mixture may contain unmodified protein having a molecular weight of about 20,000 Daltons, monoPEGylated protein (or "1-mer") having a molecular weight of about 40,000 Daltons, diPEGylated protein (or 2-mer") having a molecular weight of about 60,000 Daltons.

While this approach can be used to separate PEG conjugates having different molecular weights, this approach is generally ineffective for separating positional isomers having different polymer attachment sites within the GM-CSF moiety. For example, gel filtration chromatography can be used to separate from each other mixtures of 1-mers, 2-mers, 3-mers, and so forth, although each of the recovered PEG-mer compositions may contain PEGs attached to different reactive amino groups (e.g., lysine residues) within GM-CSF moiety.

Gel filtration columns suitable for carrying out this type of separation include Superdex™ and Sephadex™ columns available from Amersham Biosciences (Piscataway, NJ). Selection of a particular column will depend upon the desired fractionation range desired. Elution is generally carried out using a suitable buffer, such as phosphate, acetate, or the like. The collected fractions may be analyzed by a number of different methods, for example, (i) absorbance at 280 nm for protein content, (ii) dye-based protein analysis using bovine serum albumin as a standard, (iii) iodine testing for PEG content (Sims et al. (1980) Anal. Biochem, 107:60-63), (iv) sodium dodecyl sulfate polyacrylamide gel electrophoresis (SDS PAGE), followed by staining with barium iodide, and higher performance liquid chromatography.

Separation of positional isomers can be carried out by reverse phase chromatography using reverse phase-high performance liquid chromatography (RP-HPLC) methods using, for example, a C18 column or C3 column (Amersham Biosciences or Vydac) or by ion exchange chromatography using an ion exchange column, e.g., a Sepharose™ ion exchange column available from Amersham Biosciences. Either approach can be used to separate polymer-active agent isomers having the same molecular weight (positional isomers).

The compositions are preferably substantially free of proteins that do not have GM-CSF activity. In addition, the compositions preferably are substantially free of all other noncovalently attached water-soluble polymers. In some circumstances, however, the composition can contain a mixture of water-soluble polymer-(GM-CSF) moiety conjugates and unconjugated GM-CSF.

Optionally, the composition of the invention further comprises a pharmaceutically acceptable excipient. If desired, the pharmaceutically acceptable excipient can be added to a conjugate to form a composition.

Exemplary pharmaceutically acceptable excipients include, those selected from the group consisting of carbohydrates, inorganic salts, antimicrobial agents, antioxidants, surfactants, buffers, acids, bases, and combinations thereof.

A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and alditols, such as mannitol, xylitol, maltitol, lactitol, xylitol, sorbitol (glucitol), pyranosyl sorbitol, myoinositol.

The excipient can also include an inorganic salt or buffer such as citric acid, sodium chloride, potassium chloride, sodium sulfate, potassium nitrate, sodium phosphate monobasic, sodium phosphate dibasic, and combinations thereof.

The composition can also include an antimicrobial agent for preventing or deterring microbial growth. Examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the composition as well. Antioxidants are used to prevent oxidation, thereby preventing the deterioration of the conjugate or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant can be present as an excipient. Exemplary surfactants include: polysorbates, such as "Tween 20" and "Tween 80," and pluronics such as F68 and F88 (both of which are available from BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; and chelating agents, such as EDTA, zinc and other such suitable cations.

Acids or bases can be present as an excipient in the composition. Nonlimiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of the conjugate (i.e., the conjugate formed between the active agent and the polymeric reagent) in the composition will vary depending on a number of factors, but will optimally be a therapeutically effective amount when the composition is stored in a unit dose container (e.g., a vial). In addition, the pharmaceutical preparation can be housed in a syringe. A therapeutically effective amount can be determined experimentally by repeated administration of increasing amounts of the conjugate in order to determine which amount produces a clinically desired endpoint.

The amount of any individual excipient in the composition will vary depending on the activity of the excipient and particular needs of the composition. Typically, the optimal amount of any individual excipient is determined through routine experimentation, i.e., by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99% by weight, preferably from about 5% to about 98% by weight, more preferably from about 15 to about 95% by weight of the excipient, with concentrations less than 30% by weight most preferred.

These foregoing pharmaceutically acceptable excipients along with other excipients are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D.C., 2000.

A method for making a conjugate is also provided, the method comprising contacting, under conjugation conditions, a GM-CSF moiety with a polymeric reagent. As provided herein, the method does not necessarily involve carrying out protecting and deprotecting steps. The Experimental section below provides exemplary approaches for making conjugates. Once a conjugate is prepared, a pharmaceutically acceptable excipient can be added to the conjugate to provide a pharmaceutical composition.

The pharmaceutical compositions encompass all types of formulations and in particular those that are suited for injection, e.g., powders or lyophilates that can be reconstituted as well as liquids. Examples of suitable diluents for reconstituting solid compositions prior to injection include bacteriostatic water for injection, dextrose 5% in water, phosphate-buffered saline, Ringer's solution, saline, sterile water, deionized water, and combinations thereof. With respect to liquid pharmaceutical compositions, solutions and suspensions are envisioned.

Suitable formulation types for parenteral administration include ready-for-injection solutions, dry powders for combination with a solvent prior to use, suspensions ready for injection, dry insoluble compositions for combination with a vehicle prior to use, and emulsions and liquid concentrates for dilution prior to administration, among others.

In addition, the conjugate can be administered to a patient undergoing bone marrow transplantation (such as a patient suffering from acute myelogenous leukemia), wherein administration occurs prior to, simultaneously with, or after the bone marrow transplant (either autologous or allogenic). Furthermore, the conjugate can be used in the treatment of cancers via enhancement of the cytotoxic activity of peripheral monocytes and lymphocytes, mucositis, stomatitis, diarrhea, wound healing, pulmonary alveolar proteinosis, and hypercholesterolemia. Finally, the conjugates can also be used as a vaccine adjuvant.

The actual dose to be administered will vary depending upon the age, weight, and general condition of the subject as well as the severity of the condition being treated, the judgment of the health care professional, and conjugate being administered. Therapeutically effective amounts are known to those skilled in the art and/or are described in the pertinent reference texts and literature. Generally, on a weight basis, a therapeutically effective amount will range from about 0.001 mg to 100 mg, preferably in doses from 0.01 mg/day to 75 mg/day, and more preferably in doses from 0.10 mg/day to 50 mg/day. On an activity basis, corresponding doses based on international units of activity can be calculated by one of ordinary skill in the art.

The unit dosage of any given conjugate (again, preferably provided as part of a pharmaceutical composition) can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, without limitation, administration five times a day, four times a day, three times a day, twice daily, once daily, three times weekly, twice weekly, once weekly, twice monthly, once monthly, and any combination thereof. Once the clinical endpoint has been achieved, dosing of the composition is halted.

### EXPERIMENTAL

The practice of the invention will employ, unless otherwise indicated, conventional techniques of organic synthesis, which are within the skill of the art. Such techniques are fully explained in the literature. Reagents and materials are commercially available unless specifically stated to the contrary. See, for example, J. March, Advanced Organic Chemistry: Reactions Mechanisms and Structure, 4th Ed. (New York: Wiley-Interscience, 1992), *supra*.

In the following examples, efforts have been made to ensure accuracy with respect to numbers used (e.g., amounts, temperatures, etc.) but some experimental error and deviation should be accounted for. Unless indicated otherwise, temperature is in degrees C and pressure is at or near atmospheric pressure at sea level.

Although other abbreviations known by one having ordinary skill in the art will be referenced, other reagents and materials will be used, and other methods known by one having ordinary skill in the art will be used, the following list and methods description is provided for the sake of convenience.

ABBREVIATIONS:

NaCNBH₃ sodium cyanoborohydride

HCl hydrochloric acid

HEPES 4-(2-hydroxyethyl)-1-piperazineethanesulfonic acid

K or kDa kiloDaltons

SEC Size exclusion chromatography

HPLC high performance liquid chromatography

SDS-PAGE sodium dodecyl sulfate-polyacrylamide gel electrophoresis

SDS-PAGE Analysis

Samples indicated to be were analyzed by sodium dodecyl sulfate-polyacrylamide gel electrophoresis (SDS-PAGE) using a Bio-Rad system (Mini-PROTEAN ID Precast Gel Electrophoresis System). Samples were mixed with sample buffer. Then, the prepared samples were loaded onto a gel and run for approximately thirty minutes.

SEC-HPLC Analysis

Size exclusion chromatography (SEC-HPLC) analysis was performed on an Agilent 1100 HPLC system (Agilent). Samples were analyzed using a Shodex protein KW-804 column (300 x 8 mm, Phenomenex), at pH 7.4. The flow rate for the column was 0.5 mL/minute. Eluted protein and PEG-protein conjugates were detected using UV at 280nm.

Anion-Exchange Chromatography

A HiTrap Q Sepharose HP anion exchange column (Amersham Biosciences) was used with the AKTAprime system (Amersham Biosciences) to purify the PEGylated GM-CSF conjugates prepared in Example 1 through 6. For each conjugate solution prepared, the conjugate solution was loaded on a column that was pre-equilibrated in 20 mM Tris buffer, pH 7.5 (buffer A) and then washed with ten column volumes of buffer A to remove any unreacted PEG reagent. Subsequently, a gradient of buffer A with 0-100% buffer B (20mM Tris with 0.5 M NaCl buffer, pH 7.5) was raised. The eluent was monitored by UV detector at 280 nm. Any higher-mers (e.g., 3-mers, 4-mers, and so forth) eluted first, next followed by 2-mers, and then 1-mers, and finally unconjugated GM-CSF. The fractions were pooled according to the chromatogram, and the purity of the individual conjugate was determined by SEC-HPLC or SDS-PAGE.

Recombinant human GM-CSF (hGM-CSF) corresponding to the amino acid sequence of SEQ ID NO: 2. was used in Examples 1-6 and was obtained from a commercial source. A stock hGM-CSF solution was prepared by ensuring that the recombinant human GM-CSF existed in an amine-free buffer, using (if necessary) a buffer exchange technique known to those of ordinary skill in the art.

### Example 1

### PEGylation of hGM-CSF with Branched mPEG-N-Hydroxysuccinimide Derivative, 40kDa

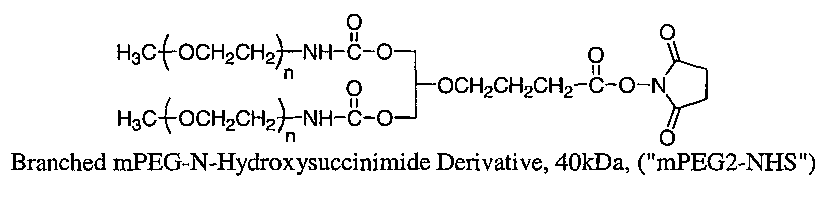

mPEG2-NHS, 40kDa, stored at -20 °C under argon, was warmed to ambient temperature. A five-fold excess (relative to the amount of hGM-CSF in a measured aliquot of the stock hGM-CSF solution) of the warmed mPEG2-NHS was dissolved in 2mM HCl to form a 10% reagent solution. The 10% reagent solution was quickly added to the aliquot of stock hGM-CSF solution (1 mg/mL in sodium phosphate buffer, pH 7.0) and mixed well. After the addition of the PEG reagent, the pH of the reaction mixture was determined and adjusted to 7.0 using conventional techniques. To allow for coupling of the mPEG2-NHS to hGM-CSF via an amide linkage, the reaction solution was placed on a Slow Speed Lab Rotator overnight to facilitate conjugation at room temperature. The reaction was quenched with Tris buffer. The conjugate solution was characterized as provided below.

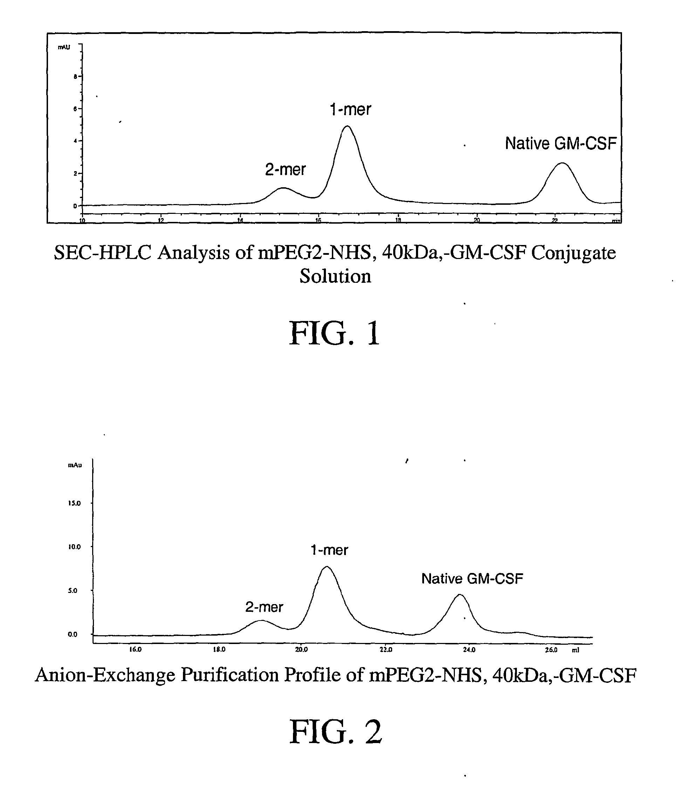

Figure 1 shows the chromatogram following the SEC-HPLC analysis of the conjugate solution. The PEGylation reaction yielded 57% 1-mer (mono-conjugate or one PEG attached to hGM-CSF) and 13% 2-mer (di-conjugate or two PEGs attached to hGM-CSF) species.

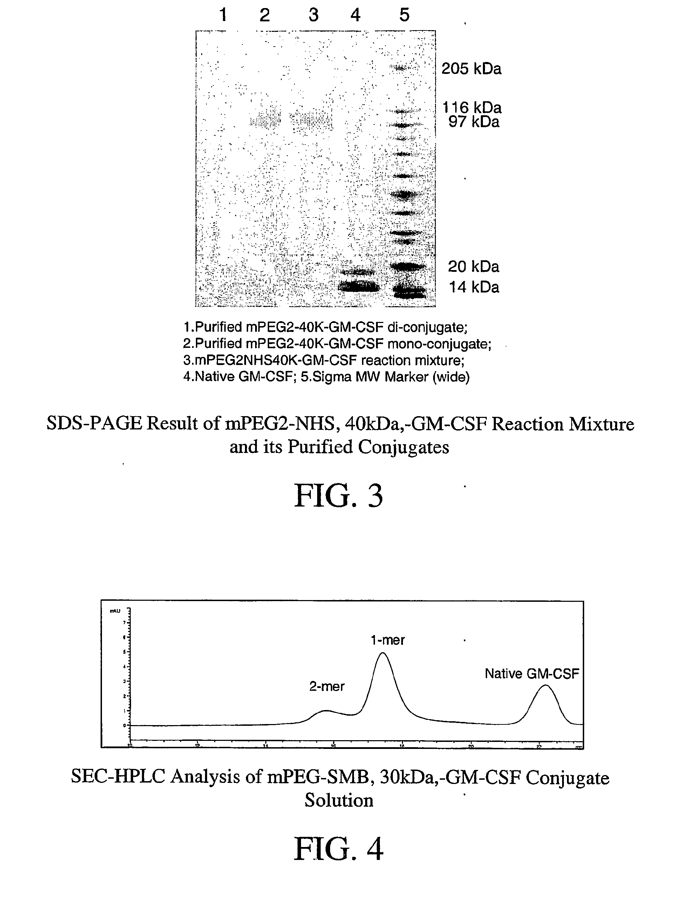

Anion-exchange chromatography was used to purify the conjugates. Figure 2 shows the chromatogram following anion-exchange purification. The conjugate fractions were collected and analyzed by SDS-PAGE (Figure 3). The purified conjugates were up to 100% pure.

Using this same approach, other conjugates can be prepared using mPEG2-NHS having other weight average molecular weights.

### Reference Example 2

### PEGylation of hGM-CSF with Linear mPEG-Succinimidyl α-Methylbutanoate Derivative, 30kDa

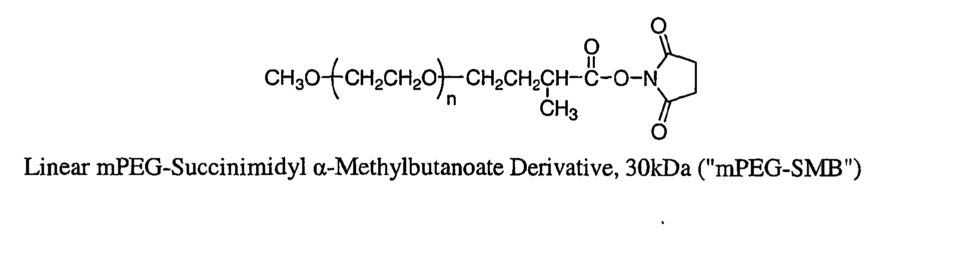

mPEG-SMB, 30kDa, stored at -20 °C under argon, was warmed to ambient temperature. A ten-fold excess (relative to the amount of hGM-CSF in a measured aliquot of the stock hGM-CSF solution) of the warmed mPEG-SMB was dissolved in 2 mM HCl to form a 10% reagent solution. The 10% reagent solution was quickly added to the aliquot of stock hGM-CSF solution (1 mg/mL in sodium phosphate buffer, pH 7.0) and mixed well. After the addition of the mPEG-SMB, the pH of the reaction mixture was determined and adjusted to 7.0 using conventional techniques. To allow for coupling of the mPEG-SMB to hGM-CSF via an amide linkage, the reaction solution was placed on a Slow Speed Lab Rotator overnight to facilitate conjugation at room temperature. The reaction was quenched with Tris buffer. The conjugate solution was characterized as provided below.

Figure 4 shows the SEC-HPLC chromatogram of the conjugate solution. The SEC-HPLC analysis reveals the PEGylation reaction yielded 58% 1-mer (mono-conjugate or one PEG attached to hGM-CSF) and 14% 2-mer (di-conjugate or two PEGs attached to hGM-CSF) species. An anion-exchange chromatography method using Q Sepharose High Performance and Tris buffer was also used to purify the conjugates. The separation profile of the conjugate species was similar to that shown in Figure 2.

Using this same approach, other conjugates can be prepared using mPEG-SMB having other weight average molecular weights.

### Reference Example 3

### PEGylation of hGM-CSF with mPEG-Piperidone, 20kDa

mPEG-Piperidone (mPEG-PIP) having a molecular weight of 20,000 Daltons is obtained from Nektar Therapeutics (Huntsville, AL). The basic structure of the polymeric reagent is provided below:

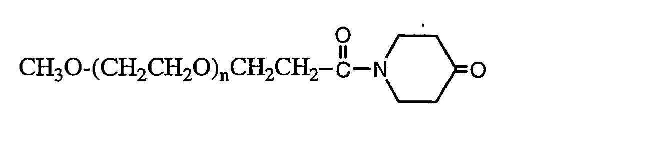

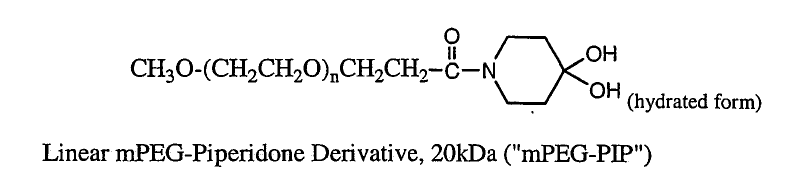

mPEG-PIP, 20kDa, stored at -20 °C under argon, was warmed to ambient temperature. A fifty to one hundred-fold excess (relative to the amount of hGM-CSF in a measured aliquot of the stock hGM-CSF) of the warmed mPEG-PIP was dissolved in 10mM sodium phosphate (pH 7.0) to form a 10% reagent solution. The 10% reagent solution was quickly added to the aliquot of stock hGM-CSF solution (1 mg/mL in sodium phosphate buffer, pH 7.0) and mixed well. After the addition of the mPEG-PIP, the pH of the reaction mixture was determined and adjusted to 7.0 using conventional techniques, followed by mixing for thirty minutes. A reducing agent, sodium cyanoborohydride, was then added to make 13 mM NaCNBH₃. The reaction solution was placed on a Slow Speed Lab Rotator overnight to facilitate conjugation at room temperature. The reaction was quenched with Tris buffer. The conjugate solution was characterized as provided below.

The PEGylation reaction yielded over 30% of 1-mer (mono-conjugate or one PEG attached to hGM-CSF). Due to the high selectivity of the PEG ring structure, little 2-mer was resulted.

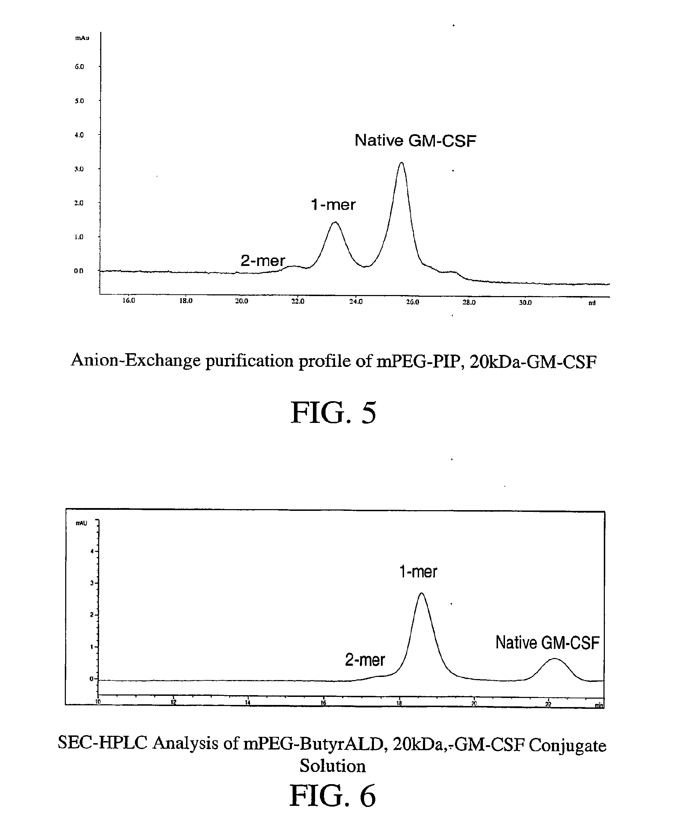

An anion-exchange chromatography method was also used to purify the conjugates. Figure 5 depicts the anion-exchange purification profile.

### Reference Example 4

### PEGylation of hGM-CSF with Linear mPEG-Butyraldehyde Derivative, 20kDa

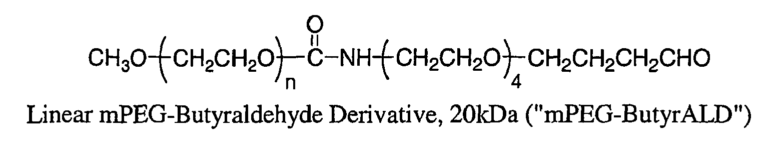

mPEG-ButyrALD, 20kDa, stored at -20 °C under argon, was warmed to ambient temperature. A thirty-fold excess (relative to the amount of hGM-CSF in a measured aliquot of the stock hGM-CSF) of the warmed mPEG-ButryALD was dissolved in Milli-Q H₂O to form a 10% reagent solution. The 10% reagent solution was quickly added to the aliquot of stock hGM-CSF solution (1 mg/mL in sodium phosphate buffer, pH 7.0) and mixed well. After the addition of the mPEG-ButryALD, the pH of the reaction mixture was determined and adjusted to 6.0 using conventional techniques, followed by mixing for thirty minutes. A reducing agent, sodium cyanoborohydride was then added to make 9 mM NaCNBH₃. The reaction solution was placed on a Slow Speed Lab Rotator overnight to facilitate conjugation at room temperature. The reaction was quenched with Tris buffer. The conjugate solution was characterized as provided below.

The aldehyde group of mPEG-ButyrALD can react with the primary amines associated with hGM-CSF and covalently bond to them via secondary amine upon reduction by a reducing reagent such as sodium cyanoborohydride. Because the PEGylation reaction was carried at pH 6.0, attachment of the PEG derivative to hGM-CSF was more selective to the N-terminal. Figure 6 shows the SEC-HPLC chromatogram of the conjugate solution. The PEGylation reaction yielded 75% 1-mer (one PEG attached to hGM-CSF or monoPEGylated) and 4% 2-mer (di-conjugate or two PEGs attached to hGM-CSF) species. An anion-exchange chromatography method using Q Sepharose High Performance and Tris buffer was also used to purify the conjugates. The separation profile of the conjugate species was similar to that shown in Figure 5.

Using this same approach, other conjugates can be prepared using mPEG-ButyrALD having other weight average molecular weights.

### Reference Example 5

### PEGylation of GM-CSF with Linear mPEG-Butyraldehyde Derivative, 30kDa

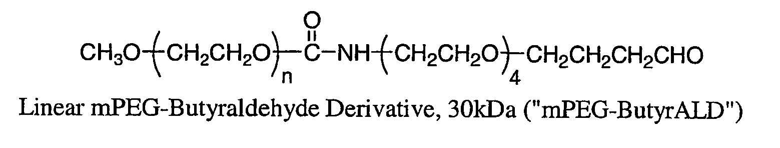

mPEG-ButyrALD, 30kDa, stored at -20 °C under argon, was warmed to ambient temperature. A thirty-fold excess (relative to the amount of hGM-CSF in a measured aliquot of the stock hGM-CSF) of the warmed mPEG-ButryALD was dissolved in Milli-Q H₂O to form a 10% reagent solution. The 10% reagent solution was quickly added to the aliquot of stock hGM-CSF solution (1 mg/mL in sodium phosphate buffer, pH 7.0) and mixed well. After the addition of the mPEG-ButryALD, the pH of the reaction mixture was determined and adjusted to 6.0 using conventional techniques, followed by mixing for thirty minutes. A reducing agent, sodium cyanoborohydride was then added to make 9 mM NaCNBH_{3.} The reaction solution was placed on a Slow Speed Lab Rotator overnight to facilitate conjugation at room temperature. The reaction was quenched with Tris buffer. The conjugate solution was characterized as provided below.

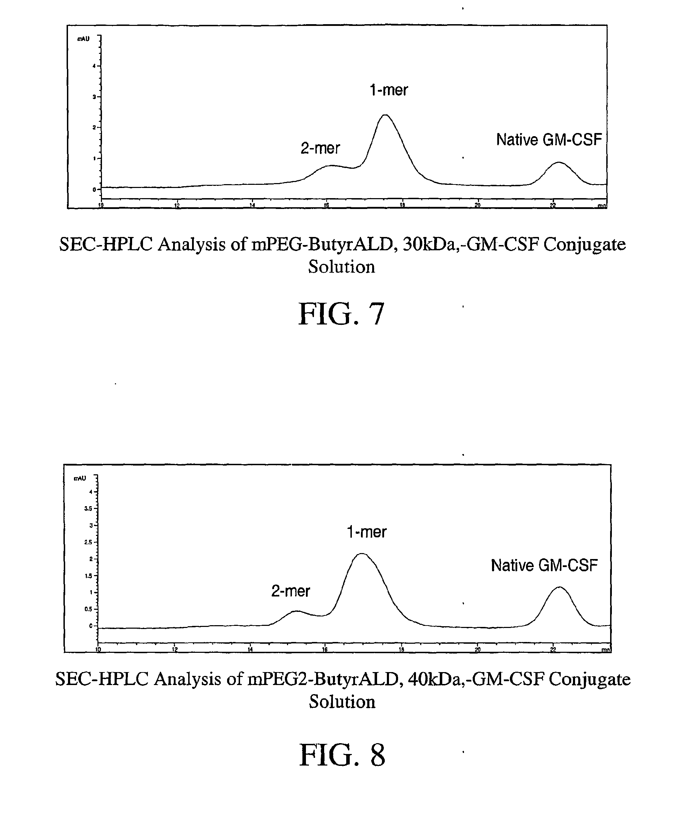

The aldehyde group of mPEG-ButyrALD can react with the primary amines associated with hGM-CSF and covalently bond to them via secondary amine upon reduction by a reducing reagent such as sodium cyanoborohydride. Because the PEGylation reaction was carried at pH 6.0, attachment of the PEG derivative to hGM-CSF was more selective to the N-terminal. Figure 7 shows the SEC-HPLC chromatogram of the conjugate solution. The PEGylation reaction yielded 63% 1-mer (one PEG attached to hGM-CSF or monoPEGylated) and 20% 2-mer (di-conjugate or two PEGs attached to hGM-CSF) species. An anion-exchange chromatography method using Q Sepharose High Performance and Tris buffer was also used to purify the conjugates. The separation profile of the conjugate species was similar to that shown in Figure 2.

Using this same approach, other conjugates can be prepared using mPEG-ButyrALD having other weight average molecular weights.

### Example 6

### PEGylation of hGM-CSF with Branched mPEG-Butyraldehyde Derivative, 40kDa

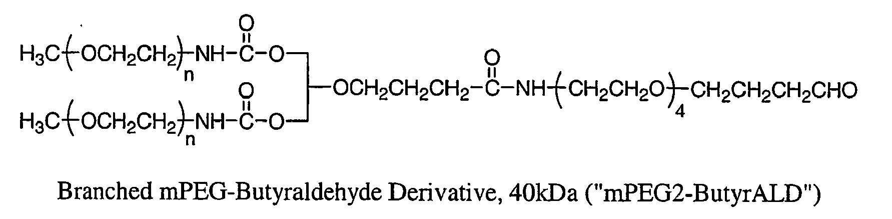

mPEG2-ButyrALD, 40kDa, stored at -20 °C under argon, was warmed to ambient temperature. A thirty-fold excess (relative to the amount of hGM-CSF in a measured aliquot of the stock hGM-CSF) of the warmed mPEG2-ButryALD was dissolved in Milli-Q H₂O to form a 10% reagent solution. The 10% reagent solution was quickly added to the aliquot of stock hGM-CSF solution (1 mg/mL in sodium phosphate buffer, pH 7.0) and mixed well. After the addition of the mPEG2-ButryALD, the pH of the reaction mixture was determined and adjusted to 6.0 using conventional techniques, followed by mixing for thirty minutes. A reducing agent, sodium cyanoborohydride was then added to make 9 mM NaCNBH_{3.} The reaction solution was placed on a Slow Speed Lab Rotator overnight to facilitate conjugation at room temperature. The reaction was quenched with Tris buffer. The conjugate solution was characterized as provided below.

The aldehyde group of mPEG2-ButyrALD can react with the primary amines associated with hGM-CSF and covalently bond to them via secondary amine upon reduction by a reducing reagent such as sodium cyanoborohydride. Because the PEGylation reaction was carried at pH 6.0, attachment of the PEG derivative to hGM-CSF was more selective to the N-terminal. Figure 8 shows the SEC-HPLC chromatogram of the conjugate solution. The PEGylation reaction yielded 65% 1-mer (one PEG attached to hGM-CSF or monoPEGylated) and 10% 2-mer (di-conjugate or two PEGs attached to hGM-CSF) species. An anion-exchange chromatography method using Q Sepharose High Performance and Tris buffer was also used to purify the conjugates. The separation profile of the conjugate species was similar to that shown in Figure 2.

Using this same approach, other conjugates can be prepared using mPEG2-ButyrALD having other weight average molecular weights.

### Reference Example 7

### PEGylation of GM-CSF with mPEG-SBA

mPEG-Succinimidyl butanoate having a molecular weight of 20,000 Daltons is obtained from Nektar Therapeutics, (Huntsville, AL). The basic structure of the polymer reagent is provided below:

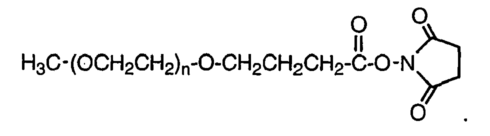

GM-CSF is dissolved in deionized water, to which is added triethylamine to raise the pH to 7.2-9. To this solution is then added a 1.5 to 10-fold molar excess of mPEG-SBA. The resulting mixture is stirred at room temperature for several hours.

The reaction mixture is analyzed by SDS-PAGE to determine the degree of PEGylation of the protein.

### Reference Example 8

### Conjugation of Cysteine-Inserted GM-CSF with mPEG-MAL, 20K

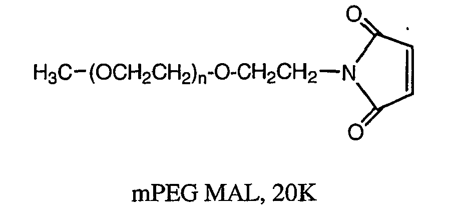

GM-CSF is inserted with one or more cysteine residues according to the process described in WO 90/12874.

mPEG-MAL, 20K, stored at -20 °C under argon, is warmed to ambient temperature. A five- to twenty-fold excess of the warmed mPEG-MAL, 20K, is dissolved in deionized water to make a 10% mPEG MAL solution. The mPEG MAL solution is quickly added to an aliquot of stock GM-CSF solution (1mg/mL in 50 mM HEPES, pH 7.0) and is mixed well. After one hour of reaction at room temperature, the reaction vial is transferred to the cold room and the reaction is allowed to proceed overnight at 4 °C on Rotomix (slow speed, Thermolyne).

The conjugate mixture is purified using gel filtration chromatography. A size exclusion chromatography method is developed for analyzing the reaction mixtures, and the final products. SDS-PAGE analysis is also used for the characterization of the samples.

### Reference Example 9

### Conjugation of GM-CSF with mPEG-MAL, 30K

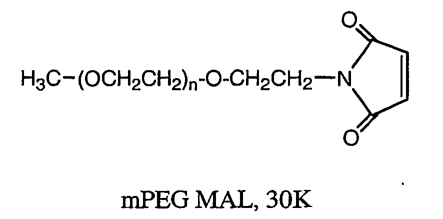

GM-CSF is inserted with one or more cysteine residues according to the process described in WO 90/12874.

mPEG-MAL, 30K, stored at -20 °C under argon, is warmed to ambient temperature. A five- to twenty-fold excess of the warmed mPEG-MAL, 30K, is dissolved in deionized water to make a 10% mPEG MAL solution. The mPEG MAL solution is quickly added to an aliquot of stock GM-CSF solution (1mg/mL in 50 mM HEPES, pH 7.0) and is mixed well. After one hour of reaction at room temperature, the reaction vial is transferred to the cold room and the reaction is allowed to proceed overnight at 4 °C on Rotomix (slow speed, Thermolyne).

The conjugate mixture is purified using gel filtration chromatography. A size exclusion chromatography method is developed for analyzing the reaction mixtures, and the final products. SDS-PAGE analysis is also used for the characterization of the samples.

### Example 10

### In-vitro Activity of Exemplary (GM-CSF)-PEG Conjugates

The in-vitro activities of the conjugates described in Examples 1-6 are determined. All of the conjugates tested are bioactive.

### Examples 11-19

Each of Examples 1-9 is replicated except that the GM-CSF moiety of SEQ ID NO: 2 is replaced with SEQ ID NO: 1 GM-CSF.

### Example 20

### In-vitro Activity Conjugates

The in-vitro activities of the conjugates described in Examples 11-19 are determined. All of the conjugates tested are bioactive.

### SEQUENCE LISTING

| SEQ ID NO: 1 | | | |
|---|---|---|---|
| APARSPSPST | QPWEHVNAIQ | EARRLLNLSR | DTAAEMNETV |
| EVISEMFDLQ | EPTCLQTRLE | LYKQGLRGSL | TKLKGPLTMM |
| ASHYKQHCPP | TPETSCATQI | ITFESFKENL | KDFLLVIPFD |
| CWEPVQE | | | |
| | | | |

| SEQ ID NO: 2 | | | |
|---|---|---|---|
| APARSPSPST | QPWEHVNAIQ | EALRLLNLSR | DTAAEMNETV |
| EVISEMFDLQ | EPTCLQTRLE | LYKQGLRGSL | TKLKGPLTMM |
| ASHYKQHCPP | TPETSCATQI | ITFESFKENL | KDFLLVIPFD |
| CWEPVQE | | | |
| | | | |

| SEQ ID NO: 3 | | | |
|---|---|---|---|
| APARSPSPST | QPWEHVNAIQ | EARLLLNLSR | DTAAEMNETV |
| EVISEMFDLQ | EPTCLQTRLE | LYKQGLRGSL | TKLKGPLTMM |
| ASHYKQHCPP | TPETSCATQI | ITFESFKENL | KDFLLVIPFD |
| CWEPVQE | | | |

## Claims

1. A pharmaceutical composition comprising:
(i) a conjugate comprising a human GM-CSF covalently attached, either directly or through a spacer moiety comprised of one or more atoms, to a water-soluble polymer, having the following structure: 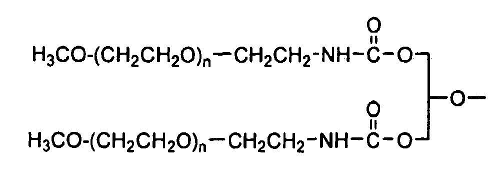 wherein each (n) is independently an integer having a value of from 3 to 4000, and having a weight-average molecular weight of greater than 5,000 Daltons; and
(ii) a pharmaceutically acceptable excipient,
wherein at least about 85% of the conjugates in the composition have from one to two polymers attached to the human GM-CSF.

2. The composition of claim 1, wherein the poly(ethylene glycol) has a total weight-average molecular weight in the range of from greater than 5,000 Daltons to about 150,000 Daltons.

3. The composition of claim 2, wherein the poly(ethylene glycol) has a total weight-average molecular weight in the range of from about 6,000 Daltons to about 100,000 Daltons.

4. The composition of claim 3, wherein the poly(ethylene glycol) has a total weight-average molecular weight in the range of from about 15,000 Daltons to about 85,000 Daltons.

5. The composition of claim 4, wherein the poly(ethylene glycol) has a total weight-average molecular weight in the range of from about 20,000 Daltons to about 85,000 Daltons.

6. The composition of claim 5, wherein the poly(ethylene glycol) has a total weight average molecular weight in the range of from about 20,000 Daltons to about 60,000 Daltons.

7. The composition of claim 1, with the proviso that the branched water-soluble polymer lacks a lysine residue used to effect branching.

8. The composition of claim 1, wherein the human GM-CSF comprises an amino acid sequence of SEQ ID NO: 1.

9. The composition of claim 6, wherein the human GM-CSF comprises an amino acid sequence of SEQ ID NO: 2.

10. The composition of claim 6, wherein the human GM-CSF is recombinantly derived.

11. The composition of claim 9, wherein the human GM-CSF is nonglycosylated.

12. The composition of claim 9, wherein the human GM-CSF is glycosylated.

13. The composition of claim 1, wherein the composition is substantially free of albumin.

14. The composition of claim 1, wherein the composition is substantially free of proteins that do not have GM-CSF activity.

15. The composition of claim 1, wherein the composition is substantially free of noncovalently attached water-soluble polymers.

16. The composition of claim 1, in lyophilized form.

17. The composition of claim 1, in liquid form.

18. The composition of claim 1, wherein at least about 90% of the conjugates in the composition have from one to two polymers attached to the human GM-CSF.

19. The composition of claim 18, wherein at least about 95% of the conjugates in the composition have from one to two polymers attached to the human GM-CSF.

20. The pharmaceutical composition of claim 1 for the use in therapy.

21. The composition of claim 20, in a form suitable for subcutaneous injection.

## Patentansprüche

1. Pharmazeutische Zusammensetzung, umfassend:
(i) ein Konjugat, umfassend einen humanen GM-CSF, der entweder direkt oder durch einen Spacerrest, der ein oder mehrere Atom(e) umfasst, kovalent an ein wasserlösliches Polymer gebunden ist, welches die folgende Struktur aufweist 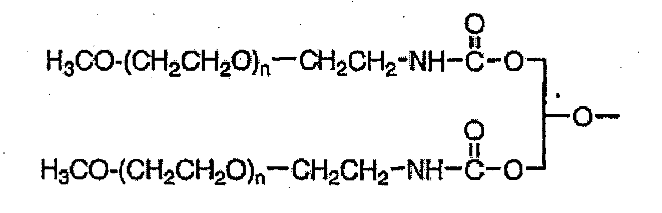 worin jedes (n) unabhängig voneinander eine ganze Zahl ist, die einen Wert von 3 bis 4.000 aufweist, und die ein gewichtsmittleres Molekulargewicht von größer als 5.000 Dalton aufweist; und
(ii) ein pharmazeutisch akzeptabler Hilfsstoff,
wobei mindestens etwa 85% der Konjugate in der Zusammensetzung von einem bis zwei Polymere aufweisen, die an den humanen GM-CSF gebunden sind.

2. Zusammensetzung nach Anspruch 1, wobei das Polyethylenglykol ein gesamtes gewichtsmittleres Molekulargewicht im Bereich von größer als 5.000 Dalton bis etwa 150.000 Dalton aufweist.

3. Zusammensetzung nach Anspruch 2, wobei das Polyethylenglykol ein gesamtes gewichtsmittleres Molekulargewicht im Bereich von etwa 6.000 Dalton bis etwa 100.000 Dalton aufweist.

4. Zusammensetzung nach Anspruch 3, wobei das Polyethylenglykol ein gesamtes gewichtsmittleres Molekulargewicht im Bereich von etwa 15.000 Dalton bis etwa 85.000 Dalton aufweist.

5. Zusammensetzung nach Anspruch 4, wobei das Polyethylenglykol ein gesamtes gewichtsmittleres Molekulargewicht im Bereich von etwa 20.000 Dalton bis etwa 85.000 Dalton aufweist.

6. Zusammensetzung nach Anspruch 5, wobei das Polyethylenglykol ein gesamtes gewichtsmittleres Molekulargewicht im Bereich von etwa 20.000 Dalton bis etwa 60.000 Dalton aufweist.

7. Zusammensetzung nach Anspruch 1, mit der Maßgabe, dass das verzweigte wasserlösliche Polymer keinen Lysinrest enthält, der verwendet wird, um eine Verzweigung zu bewirken.

8. Zusammensetzung nach Anspruch 1, wobei der humane GM-CSF eine Aminosäuresequenz der SEQ ID NO: 1 umfasst.

9. Zusammensetzung nach Anspruch 6, wobei der humane GM-CSF eine Aminosäuresequenz der SEQ ID NO: 2 umfasst.

10. Zusammensetzung nach Anspruch 6, wobei der humane GM-CSF rekombinant gewonnen wird.

11. Zusammensetzung nach Anspruch 9, wobei der humane GM-CSF nichtglykosyliert ist.

12. Zusammensetzung nach Anspruch 9, wobei der humane GM-CSF glykosyliert ist.

13. Zusammensetzung nach Anspruch 1, wobei die Zusammensetzung im Wesentlichen frei von Albumin ist.

14. Zusammensetzung nach Anspruch 1, wobei die Zusammensetzung im Wesentlichen frei von Proteinen ist, die keine GM-CSF-Aktivität aufweisen.

15. Zusammensetzung nach Anspruch 1, wobei die Zusammensetzung im Wesentlichen frei von nichtkovalent gebundenen wasserlöslichen Polymeren ist

16. Zusammensetzung nach Anspruch 1 in lyophilisierter Form.

17. Zusammensetzung nach Anspruch 1 in flüssiger Form.

18. Zusammensetzung nach Anspruch 1, wobei mindestens etwa 90% der Konjugate in der Zusammensetzung von einem bis zwei Polymere aufweisen, die an den humanen GM-CSF gebunden sind.

19. Zusammensetzung nach Anspruch 18, wobei mindestens etwa 95% der Konjugate in der Zusammensetzung von einem bis zwei Polymere aufweisen, die an den humanen GM-CSF gebunden sind.

20. Pharmazeutische Zusammensetzung nach Anspruch 1 zur Verwendung in der Therapie.

21. Zusammensetzung nach Anspruch 20 in einer Form, die für subkutane Injektion geeignet ist.

## Revendications

1. Composition pharmaceutique comprenant :
(i) un conjugué comprenant un GM-CSF humain fixé par covalence, directement ou par un groupement intercalaire constitué d'un ou de plusieurs atome, à un polymère hydrosoluble, ayant la structure suivante : 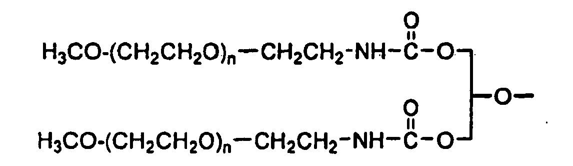 dans laquelle chaque (n) représente indépendamment un nombre entier ayant une valeur de 3 à 4000, et ayant une moyenne en poids du poids moléculaire supérieure à 5000 Daltons ; et
(ii) un excipient pharmaceutiquement acceptable,
dans laquelle une proportion d'au moins 85 % des conjugués dans la composition comporte un ou deux polymères fixés au GM-CSF humain.

2. Composition suivant la revendication 1, dans laquelle le poly(éthylèneglycol) a une moyenne en poids du poids moléculaire totale comprise dans l'intervalle de plus de 5000 Daltons à environ 150 000 Daltons.

3. Composition suivant la revendication 2, dans laquelle le poly(éthylèneglycol) a une moyenne en poids du poids moléculaire totale comprise dans l'intervalle d'environ 6000 Daltons à environ 100 000 Daltons.

4. Composition suivant la revendication 3, dans laquelle le poly(éthylèneglycol) a une moyenne en poids du poids moléculaire totale comprise dans l'intervalle d'environ 15 000 Daltons à environ 85 000 Daltons.

5. Composition suivant la revendication 4, dans laquelle le poly(éthylèneglycol) a une moyenne en poids du poids moléculaire totale comprise dans l'intervalle d'environ 20 000 Daltons à environ 85 000 Daltons.

6. Composition suivant la revendication 5, dans laquelle le poly(éthylèneglycol) a une moyenne en poids du poids moléculaire totale comprise dans l'intervalle d'environ 20 000 Daltons à environ 60 000 Daltons.

7. Composition suivant la revendication 1, sous réserve que le polymère hydrosoluble ramifié est dépourvu de résidus lysine utilisé pour effectuer la ramification.

8. Composition suivant la revendication 1, dans laquelle le GM-CSF comprend une séquence d'amino-acides de la SEQ ID NO:1.

9. Composition suivant la revendication 6, dans laquelle le GM-CSF humain comprend une séquence d'aminoacides de la SEQ ID NO:2.

10. Composition suivant la revendication 6, dans laquelle le GM-CSF humain est obtenu par recombinaison.

11. Composition suivant la revendication 9, dans laquelle le GM-CSF humain est non glycosylé.

12. Composition suivant la revendication 9, dans laquelle le GM-CSF humain est glycosylé.

13. Composition suivant la revendication 1, qui est substantiellement dépourvue d'albumine.

14. Composition suivant la revendication 1, qui est substantiellement dépourvue de protéines qui n'ont pas d'activité de GM-CSF.

15. Composition suivant la revendication 1, qui est substantiellement dépourvue de polymères hydrosolubles fixés de manière non covalente.

16. Composition suivant la revendication 1 sous forme lyophilisée.

17. Composition suivant la revendication 1, sous forme liquide.

18. Composition suivant la revendication 1, dans laquelle une proportion d'au moins 90 % des conjugués dans la composition comporte un ou deux polymères fixés au GM-CSF humain.

19. Composition suivant la revendication 18, dans laquelle une proportion d'au moins environ 95 % des conjugués dans la composition comportant un ou deux polymères fixés au GM-CSF humain.

20. Composition pharmaceutique suivant la revendication 1, destinée à être utilisée en thérapie.

21. Composition suivant la revendication 20, sous une forme convenable pour l'injection sous-cutanée.
